(19) Europäisches Patentamt
European Patent Office
Office européen des brevets

(11) Veröffentlichungsnummer: **0 157 170**
**A2**

# (12) EUROPÄISCHE PATENTANMELDUNG

(21) Anmeldenummer: **85102127.9**

(22) Anmeldetag: **27.02.85**

(51) Int. Cl.[4]: **C 12 N 11/02**, C 12 N 9/96

(30) Priorität: **07.03.84 DE 3408299**

(43) Veröffentlichungstag der Anmeldung: **09.10.85 Patentblatt 85/41**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Egerer, Peter, Dr., Claudiusweg 7, D-5600 Wuppertal 1 (DE)**
Erfinder: **Crueger, Wulf, Dr., Ruhrstrasse 35, D-4006 Erkrath 2 (DE)**
Erfinder: **Gölker, Christian, Dr., Am Rohm 35, D-5600 Wuppertal 1 (DE)**

## (54) Verfahren zur Immobilisierung von Zellen und Enzymen.

(57) Zellen und/oder Enzyme werden durch Flockung und Härtung mit einem bi- oder polyfunktionellen Aldehyd in Gegenwart niedermolekularer Monoamine immobilisiert. Die immobilisierten Zellen und Enzyme können als Biokatalysatoren verwendet werden.

EP 0 157 170 A2

BAYER AKTIENGESELLSCHAFT 5090 Leverkusen, Bayerwerk

Konzernverwaltung RP
Patentabteilung Si/Ke-c

## Verfahren zur Immobilisierung von Zellen und Enzymen

Gefunden wurde ein Verfahren zur Immobilisierung von Zellen und Enzymen durch Flockung und Härtung mit einem bi- oder polyfunktionellen Aldehyd, welches dadurch gekennzeichnet ist, daß die Immobilisierung in Gegenwart niedermolekularer, wasserlöslicher Monoamine und gegebenenfalls in Gegenwart von Ammoniak durchgeführt wird.

Aus dem Europäischen Patent 53764 ist bereits ein Verfahren zur Immobilisierung von Enzymen bekannt, bei denen eine Flockung in Gegenwart bifunktioneller Aldehyde, insbesondere Glutaraldehyd, durchgeführt wird. Bei diesem Verfahren bewirkt der Glutaraldehyd jedoch eine beträchtliche Verringerung der Enzymaktivität. Diesen Nachteil vermeidet das erfindungsgemäße Verfahren.

Das erfindungsgemäße Verfahren beruht auf der Verwendung niedermolekularer, wasserlöslicher Monoamin-Verbindungen bei der Immobilisierung, um den inaktivierenden Einfluß von Aldehyden zu mindern. Dabei wirkt

die Monoamin-Verbindung vermutlich als chemischer Puffer, indem sie den Aldehyd reversibel als Schiff-Base bindet. Die Monoamin-Verbindungen werden während der Flockung nicht mit im immobilisierten Biomaterial eingeschlossen, sondern beim Waschvorgang zusammen mit überschüssigem Aldehyd entfernt.

Geeignete Monoamin-Verbindungen sind wasserlösliche Amine und deren Derivate, insbesondere Schwefelsäure-Mono(2-Aminoethyl)Ester (I), 2-Aminoethanol (II), Glycin (III) oder Tris(hydroxymethyl)aminomethan (IV) der nachstehenden Formeln:

$NH_2-CH_2-CH_2OSO_3H$ (I)

$NH_2-CH_2-CH_2-OH$ (II)

$NH_2-CH_2-COOH$ (III)

$(HO-CH_2)_3-C-NH_2$ (IV)

Unter den für die Durchführung des erfindungsgemäßen Verfahrens infrage kommenden Aldehyden ist Glutaraldehyd besonders geeignet.

Gemäß der Erfindung werden bei der Zugabe von Glutaraldehyd-haltiger Lösung die genannten Monoamin-Derivate, in fester oder gelöster Form zugefügt. Dies kann vor, nach, oder gemeinsam mit dem Glutaraldehyd geschehen. Die geflockten Zellen werden von der Mutterlauge durch

Sedimentation oder Zentrifugation getrennt und mehrere Male mit Wasser gewaschen. Während dieser Waschschritte, werden sowohl überschüssiger Glutaraldehyd als auch die schützende Amino-Verbindung nahezu vollständig entfernt. Besonders für diese Aufarbeitungszeit ist der Schutz der Enzymaktivität vor Glutaraldehyd durch die Amino-Verbindung von entscheidender Bedeutung, wie anhand der vergleichenden nachfolgenden Beispiele eindeutig belegt werden kann.

In Tabelle 1 sind eine Reihe von Aminoverbindungen als Beispiel aufgeführt und in ihrer Effektivität zum Schutz der Sucrose-Mutase aus Protaminobacter rubrum gegenüber Glutaraldehyd verglichen.

In Abbildung 1 ist die Konzentrationsabhängigkeit der schützenden Wirkung von Aminoverbindungen am Beispiel der Verbindungen (I) und (II) gezeigt. Die maximale spezifische Aktivität ist zwischen 0,1 M und 0,2 M Aminoverbindung beobachtbar. Die spezifischen Aktivitäten einer großen Anzahl von Immobilisierungsexperimenten mit Aminoverbindung (I) und (IV) sind den spezifischen Aktivitäten ohne Zusatz in Abb. 2 und Abb. 3 gegenübergestellt. Die spezifischen Aktivitäten sind jeweils gegen die Volumenaktivität der Fermentationsbrühe aufgetragen. Die Mittelwerte aller Experimente der Abb. 2 und 3 sind 57 U/g für die Experimente ohne Zusatz, jedoch 224 U/g für Experimente mit Aminoverbindung (IV) und 218 U/g für Experimente mit Aminoverbindung (I). Diese Statistik belegt den schützenden Einfluß der Aminoverbindungen gegenüber Glutaraldehyd unabhängig von der individuellen Flockungsmethode bei einer Vielzahl von Experimenten.

Die mechanischen Stabilitäten der immobilisierten Präparate mit und ohne Zusatz einer der obigen Aminoverbindungen sind miteinander vergleichbar.

Die erfindungsgemäße schützende Aminoverbindung kann auch direkt bei der Fermentation der Zellen mitgebildet, also durch Fermentation in situ hergestellt werden. Zum Beispiel können auf diese Weise Aminosäureproduzenten wie Corynebacterium glutamicum, Brevibacterium flavum, Escherichia coli, Bacillus subtilis oder Arthrobacter paraffineus direkt aus der Fermentationslösung immobilisiert und geschützt durch die bei der Fermentation gebildete Aminosäure mittels eines bifunktionellen Aldehyds vernetzt werden.

Die nach dem erfindungsgemäßen Verfahren immobilisierten Zellen und Enzyme können als Biokatalysatoren zu Biotransformationen verwendet werden.

0157170

Beispiel 1

Immobilisierung von Protaminobacter rubrum in Gegenwart von Schwefelsäure-mono-(2-aminoethyl)-ester (I).

Zur Produktion von Sucrose-Mutase wird der Stamm Protaminobacter rubrum Z 12 (CBS 574.77) verwendet. Die Nährlösung besteht aus 5 % Dicksaft, 2 % Maisquellwasser und 0,05 % $(NH_4)_2HPO_4$, der pH-Wert beträgt 7,2. Mit 1 ml einer Protaminobacter rubrum-Abschwemmung werden in einem 1 l Erlenmeyerkolben 200 ml Nährlösung beimpft. Die Fermentation läuft 15 h bei 31°C auf der Rundschüttelmaschine.
1 U Sucrose-Mutase = 1µMol/min Umsatz von Sucrose zu Isomaltulose

1 l Fermenterbrühe der Aktivität 6,0 U/ml pH 5,5, wird bei 8°C unter Rühren mit 60 ml Tannin (4 %), danach mit 150 ml einer BETZ-Glutaraldehydmischung (60 g/l BETZ, 2,2 % v/v Glutaraldehyd, pH 9,0) zur vollständigen Flockung der Protaminobacter Zellen versetzt. (BETZ ist ein Warenzeichen der Betz Laboratories, Inc., Trevase, Pennsylvania. Es bezeichnet ein Epihalohydrinpolyamincopolymer mit einem Molekulargewicht unter einer Million, enthaltend etwa 0,288 Millimol Aminogruppen pro g Lösung.) Zur Vernetzung werden sofort weitere 75 ml der gleichen BETZ-Glutaraldehyd Mischung hinzugegeben. Dabei steigt der pH-Wert auf 7,2 an. Unmittelbar danach werden 30 g festes (I), entsprechend einer Endkonzentration von etwa 0,17 M eingerührt und der pH-Wert mit 1 N NaOH auf 7,2 nachgestellt. Nach Abzentrifugieren der Flocken wird der Überstand abgegossen, das Sediment

mit 1 l $H_2O$ gewaschen und über einen Büchner-Trichter möglichst trocken gesaugt. Der Filterkuchen wird mittels einer Handpresse extrudiert und über Nacht bei 40 bis 45°C getrocknet. Ein weiterer Ansatz, jedoch ohne (I), wurde identisch gefällt und aufgearbeitet. Nach dem Mahlen und Sieben weist die Mittelfraktion (0,5 bis 1,0 mm Korngröße) des Ansatzes mit (I) 231,0 U/g bei 23,3 % Aktivitätsausbeute auf, jene des Ansatzes ohne (I) 41,2 U/g bei 2,8 % Aktivitätsausbeute. Die Ausbeuten waren mit 13,1 g bzw. 13,8 g nahezu gleich (jeweils Trockengewichte).

Beispiel 2

Immobilisierung von Protaminobacter rubrum in Gegenwart verschiedener Mengen an (I).

Analog Beispiel 1 wurden Parallelansätze zu jeweils 1 l Zellbrühe (4,6 U/ml) geflockt und in Gegenwart unterschiedlicher Konzentrationen von (I) vernetzt und aufgearbeitet. Die spezifische Aktivität in Gegenwart von 0,01 M (I) war 105,6 U/g bei 7,4 % Aktivitätsausbeute, in Gegenwart von 0,1 M (I) 288,5 U/g bei 33,9 % Aktivitätsausbeute, und in Gegenwart von 0,2 M (I) 301,8 U/g bei 25,7 % Aktivitätsausbeute (vergleiche Abb. 1). Die Ausbeuten (Trockengewicht) lagen vor dem Mahlen und Sieben bei 10,2 g, 10,3 g und 10,5 g.

Beispiel 3

Immobilisierung von Protaminobacter rubrum in Gegenwart von I und Ammoniak.

Jeweils 1 l Zellsuspension der Aktivität 9,2 U/ml werden in Parallelansätzen geflockt und vernetzt. Zu 1 l Zellsuspension werden zunächst 120 ml einer 4 % Tanninlösung, danach 160 ml BETZ-Glutaraldehyd Mischung des Beispiel 1 bis zur vollständigen Flockung zugegeben. Anschließend wird die Suspension unter Rühren sofort mit weiteren 80 ml der BETZ-Glutaraldehyd Mischung, 0,2 Mol (I) und 0,2 Mol Ammoniak-Lösung versetzt und der pH-Wert auf 8,0 eingestellt. Die Aufarbeitung erfolgt wie in Beispiel 1.
Spezifische Aktivität der Sieb-Mittelfraktion: 198,4 U/g; 25,1 % Aktivitätsausbeute. Gegenüber Ansätzen ohne Zusatz von (I) oder lediglich mit (I)-Zusatz weist diese Partie eine wesentlich höhere mechanische Stabilität auf.
Analysenwerte zweier Vergleichsansätze: 0,1 M (I) 281,9 U/g, 21,4 % Aktivitätsausbeute; ohne Zusatz von Aminoverbindungen 61,4 U/g, 6,0 % Aktivitätsausbeute.
Die Trockengewichte vor Mahlen und Sieben waren nahezu gleich: 20,4 g, 20,3 g, 21,8 g.

## Beispiel 4

Immobilisierung von Serratia plymuthica in Gegenwart von (I).

Zur Produktion von Sucrose-Mutase wird der Stamm Serratia plymuthica (ATCC 15928) verwendet. Die Nährlösung besteht aus 5 % Dicksaft, 2 % Maisquellwasser und 0,05 % $(NH_4)_2HPO_4$, der pH-Wert beträgt 7,0. Mit 1 ml einer Abschwemmung werden in einem 1 l Erlenmeyerkolben 200 ml Nährlösung beimpft. Die Fermentation läuft 15 h bei 31°C auf der Rundschüttelmaschine.

1 l Fermenterbrühe, 7,2 U/ml, pH 6,5, wird bei 8°C unter Rühren mit 120 ml Tannin (4 %), danach mit 135 ml einer BETZ-Glutaraldehydmischung gemäß Beispiel 1 bis zur vollständigen Flockung versetzt. Die Suspension wird nun mit weiteren 60 ml der BETZ-Glutaraldehydmischung sowie mit 0,2 Mol der Aminoverbindung (I) versetzt, und der pH-Wert auf 6,5 nachgestellt.
Nach Aufarbeitung gemäß Beispiel 1 ergibt sich eine spezifische Aktivität der Sieb-Mittelfraktion von 171,2 U/g.
Demgegenüber weist ein Parallelansatz ohne (I)-Zusatz lediglich 69,8 U/g in der Sieb-Mittelfraktion auf.

Beispiel 5

Immobilisierung von Protaminobacter rubrum in Gegenwart von Glycin.

Die Anzucht von Protaminobacter rubrum Z 12 CBS 574.77 erfolgt wie in den Beispielen 1 bis 3. 1 l Zellsuspension der Aktivität 8,2 U/ml wird mit 120 ml einer 4 %igen Tanninlösung und 170 ml BETZ-Glutaraldehyd Mischung gemäß Beispiel 1 vollständig geflockt. Anschließend werden weitere 85 ml BETZ-Glutaraldehyd Mischung und 0,1 Mol Glycin in fester Form zugegeben und der pH-Wert auf 6,7 eingestellt. Die Aufarbeitung erfolgt wie unter Beispiel 1 angegeben.

Spezifische Aktivität: 232,0 U/g bei 10,2 % Aktivitätsausbeute und 11,5 g Trockengewicht vor Mahlen und Sieben.

Eine Vergleichspartie ohne Zugabe von Glycin vernetzt weist 29,9 U/g bei 2,0 % Aktivitätsausbeute und 13,4 g Trockengewicht vor Mahlen und Sieben auf.

Beispiel 6

Immobilisierung von Protaminobacter rubrum in Gegenwart von 2-Aminoethanol (II) oder Tris-(hydroxymethyl)-aminomethan (IV). Jeweils 1 l Fermenterlösung einer nach Beispiel 1 angezüchteten Protaminobacter rubrum Z 12 (CBS 574.77)-Kultur der Aktivität 3,4 U/ml werden jeweils mit 60 ml einer 4 %-Tanninlösung und nachfolgend mit 100 ml BETZ-Glutaraldehyd Mischung unter Rühren zur vollständigen Flockung versetzt. Zusätzlich zu weiteren 50 ml dieser BETZ-Glutaraldehyd Mischung werden einem der Ansätze 0,2 Mol 2-Aminoethanol, einem anderen Ansatz 0,2 Mol (IV) zugegeben. Die pH-Werte aller Ansätze werden danach auf pH 7,5 eingestellt und wie in Beispiel 1 aufgearbeitet.

| | ohne Zusatz | 0,2 Mol (II) | 0,2 Mol (IV) |
|---|---|---|---|
| Spezif. Aktivität U/g | 30,5 | 324,7 | 180,4 |
| Aktivitätsausbeute % | 7 | 58,5 | 23,8 |
| Trockensubstanz g | 13,4 | 11,8 | 9,1 |

Beispiel 7

Immobilisierung von Escherichia coli in Gegenwart von (I).

Zur Produktion von Penicillin-Acylase wird Escherichia coli ATCC 9637 verwendet. Die Nährlösung besteht aus 2 % Maisquellwasser plus 1 % Ammonium-Phenylacetat, der pH-Wert beträgt 6,5. Mit 1 ml einer Escherichia coli Abschwemmung werden 200 ml Nährlösung in einem 1 l Erlenmeyerkolben beimpft. Die Fermentation läuft 20 h auf einer Rundschüttelmaschine mit 290 Upm bei 31°C. Zu 500 ml Zellsuspension werden zuerst 20 ml 4 %-Tanninlösung zugetropft und dann mit Hilfe von 100 ml BETZ-Glutaraldehyd Mischung (60 g/l BETZ, 2,2 % v/v Glutaraldehyd, pH 9,0) vollständig geflockt. Nach der vollständigen Flockung wird sofort 50 % BETZ-Glutaraldehyd Überschuß zugegeben. Eine Hälfte des Ansatzes wird sofort zusätzlich zum Überschuß an BETZ-Glutaraldehyd mit festem (I) bis zur Endkonzentration 0,1 M versetzt. die andere Hälfte wird ohne (I) aufgearbeitet. Nach Abtrennen, Waschen und Extrudieren wird die Zellmasse bei 40 bis 45°C über Nacht getrocknet, anschließend gemahlen und gesiebt. Ohne (I) ergab sich bei der Spaltung von Penicillin G in 6-Aminopenicillansäure und Phenylessigsäure eine spezifische Aktivität von 2,17 Pen G-Einheiten pro Gramm trockener Katalysator, entsprechend 8,36 NIPAB-Einheiten/g.
NIPAB ist die in der Literatur eingeführte Abkürzung für 6-Nitro-3-Phenylacetamido-Benzoesäure, und eine NIPAB-Einheit ist definiert als die Aktivität

$$A = \frac{E(405\ nm)}{t\ .\ 2.99} \quad [\mu Mol/min = 1\ \underline{U}]$$

(Kutzbach, C., Rauenbusch, E. (1974) Hoppe-Seyler's Z. Physiol. Chem. <u>354</u>, 45).

Mit (I) lag die spezifische Aktivität um etwa 50 % höher bei 3,29 Penicillin-G-Einheiten pro Gramm, entsprechend 12,66 NIPAB-Einheiten/g.

Beispiel 8

Immobilisierung extracellulärer Amyloglucosidase aus Aspergillus foetidus in Gegenwart von (I).

Zur Produktion von Amyloglucosidase wird Aspergillus foetidus ATCC 14 916 verwendet. Die Nährlösung besteht aus 150 g/l Maismehl, 50 g/l Maisquellwasser und 0,2 g/l einer käuflichen rohen $\alpha$-Amylase. Der pH-Wert wird vor der Sterilisation auf 6,0 eingestellt. Die Kulturlösung wird vor der Sterilisation auf 75°C erhitzt, abgekühlt und anschließend auf die Fermentationskolben verteilt. Es folgt die Sterilisation bei 121°C über 20 min. Mit 5 ml einer Sporensuspension von Aspergillus foetidus werden in einem 1 l Erlenmeyerkolben 200 ml Nährlösung beimpft. Die Fermentation läuft 100 h auf einer Rundschüttelmaschine mit 290 Upm bei 31°C. 800 ml der abzentrifugierten Fermenterlösung werden mit 48 ml 4 % Tanninlösung tropfenweise versetzt und durch weitere Zugabe von 20 ml BETZ/Glutaraldehyd Mischung gemäß Beispiel 1 vollständig geflockt, der pH dabei auf 6,5 gehalten. Eine Hälfte wird nach sofortiger Zugabe von (I) bis zur Endkonzentration von 0,1 M, die andere Hälfte ohne (I) aufgearbeitet.

0157170

Die über Nacht bei 40 bis 45°C getrocknete, geflockte Amyloglucosidase wird gemahlen und gesiebt. Die Aktivität der trockenen Partikel wird nach Vorquellen über die Maltosespaltung zu Glucose ermittelt. Freigesetzte Glucose wird im enzymatischen Test mit Hexokinase/Glucose-6-Phosphat-Dehydrogenase bestimmt.

Spezifische Aktivität mit (I): 9,5 U/g (40°C); 3,5U/g (30°C).
" " ohne (I): 8,7 U/g (40°C); 2,8 U/g (30°C).

Tabelle 1

Vergleich verschiedener Monoaminoverbindungen hinsichtlich ihres Einflusses während der Immobilisierung von Protaminobacter rubrum.

| Zusatz | spezifische Aktivität U/g immob. Zellen |
|---|---|
| ohne | 45,2 |
| $NH_3$, 0,05 M | 24,6 |
| " 0,10 M | 25,3 |
| " 0,20 M | 23,7 |
| Schwefelsäure-mono-(2-aminoethyl)ester (I) | |
| 0,2 M | 231,0 |
| (I) + $NH_3$, jeweils 0,2 M | 198,4 |
| Tris(hydroxymethyl)-aminomethan, 0,2 M (IV) | 180,4 |
| 2-Aminoethanol, 0,2 M (II) | 324,7 |
| 2-Methylaminoethanol, 0,2 M | 25,5 |
| Triethanolamin, 0,2 M | 31,5 |
| N-Methyl-diethanolamin, 0,2 M | 30,5 |
| Glycin, 0,1 M (III) | 232,0 |

0157170

## Abbildungen

### Abb. 1

Zusatz von Schwefelsäure-mono(2-aminoethyl)ester (I) und von 2-Aminoethanol (II) während der Immobilisierung. Konzentrationsabhängigkeit des Schutzeffekts, •——• (I) o---------o (II).

Abszisse: Monoaminoverbindung, molar
Ordinate: U/g immobilisierte Zellen (Trockengewicht)

### Abb. 2

Vergleich der spezifischen Aktivitäten immobilisierter Protaminobacter rubrum-Zellen mit und ohne Schwefelsäure-mono(2-aminoethyl)ester (I) +++ ohne Zusatz, ••• mit Zusatz von (I) während der Immobilisierung.

Abszisse: U/g immobilisierte Zellen (Trockengewicht)
Ordinate: U/ml Fermenterbrühe

### Abb. 3

Vergleich der spezifischen Aktivitäten immobilisierter Protaminobacter Zellen mit ( ••• ) und ohne ( +++ ) Zusatz von Tris(hydroxymethyl)-aminomethan (IV) während der Immobilisierung.

Abszisse: U/g immobilisierte Zellen (Trockengewicht)
Ordinate: U/ml Fermenterbrühe

### Abb. 4

Vergleich immobilisierte Amyloglucosidase - Spaltung von Maltose. •——• unter Zusatz von Schwefelsäure-

mono(2-aminoethyl)ester (I). O----O ohne Zusatz immobilisiert.

100 mg trockene, immobilisierte Amyloglucosidase werden bei 30°C (untere Kurve) bzw. bei 40°C (untere Kurve) in 20 ml Acetat-Puffer, pH 4,75, mit 50 mg Maltose inkubiert.

Abszisse: Reaktionszeit in Minuten

Ordinate: mg Glucose

0157170

Patentansprüche

1. Verfahren zur Immobilisierung von Zellen und/oder Enzymen durch Flockung und Härtung mit einem bi- oder polyfunktionellem Aldehyd, dadurch gekennzeichnet, daß die Immobilisierung in Gegenwart niedermolekularer, wasserlöslicher Monoamine und gegebenenfalls in Gegenwart von Ammoniak durchgeführt wird.

2. Verfahren nach Anspruch 1, dadurch gekennzeichnet, daß als Monoamine primäre Amine verwendet werden.

3. Verfahren nach Anspruch 1, dadurch gekennzeichnet, daß als Monoamin Schwefelsäure-mono-(2-aminoethyl)-ester verwendet wird.

4. Verfahren nach Anspruch 1, dadurch gekennzeichnet, daß als Monoamin 2-Aminoethanol verwendet wird.

5. Verfahren nach Anspruch 1, dadurch gekennzeichnet, daß als Monoamin Glycin verwendet wird.

6. Verfahren nach Anspruch 1, dadurch gkennzeichnet, daß als Monoamin Tris-(hydroxymethyl)-aminomethan verwendet wird.

7. Verfahren nach Anspruch 1, dadurch gekennzeichnet, daß Zellen und/oder Enzyme von Protaminobacter rubrum immobilisiert werden.

8. Verfahren nach Anspruch 1, dadurch gekennzeichnet, daß Zellen und/oder Enzyme von Serratia plymuthica immobilisiert werden.

9. Verfahren nach Anspruch 1, dadurch gekennzeichnet, daß Zellen und/oder Enzyme von Escherichia coli immobilisiert werden.

10. Verfahren nach Anspruch 1, dadurch gekennzeichnet, daß Zellen und/oder Enzyme von Aspergillus foetidus immobilisiert werden.

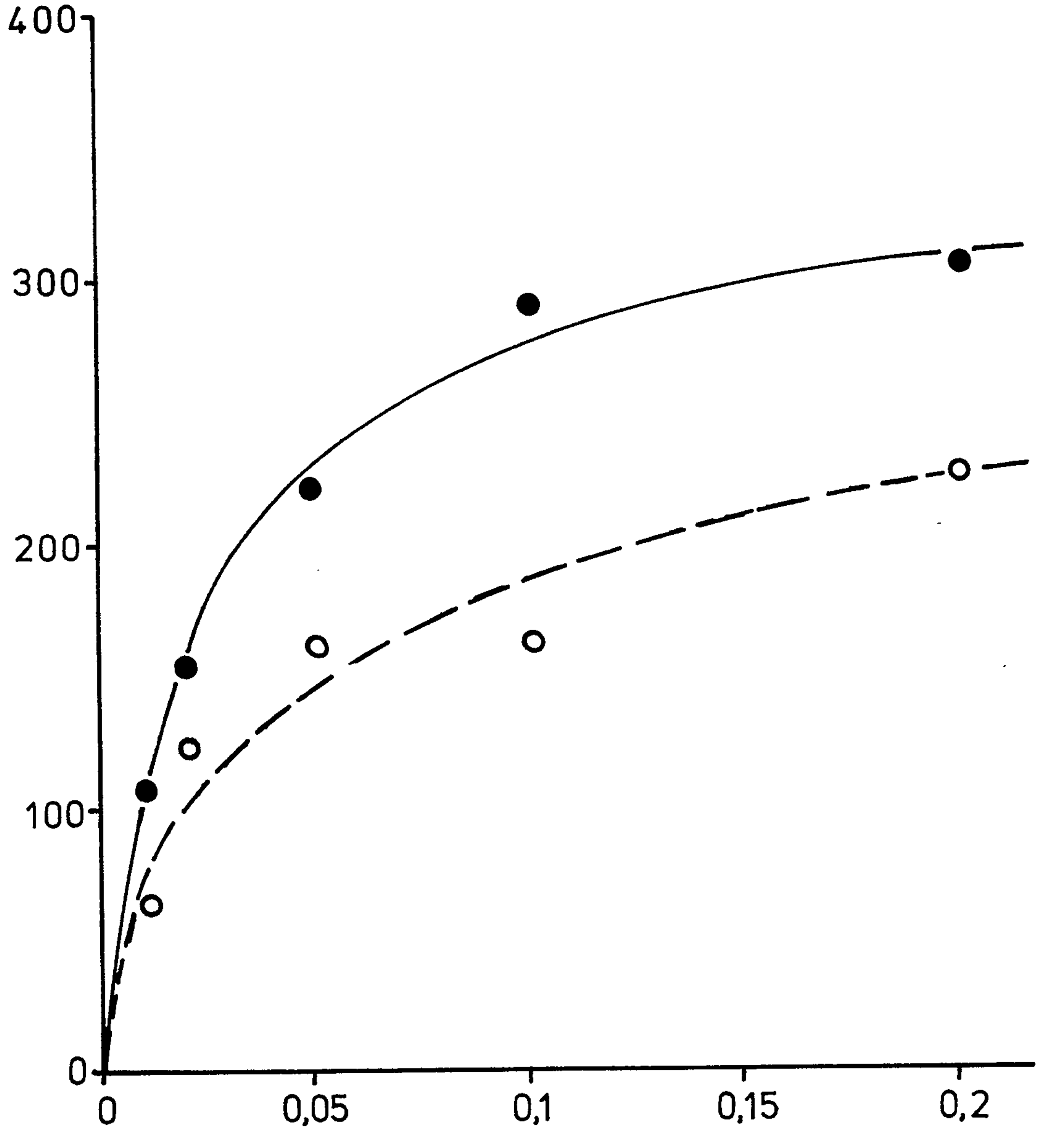


FIG. 1

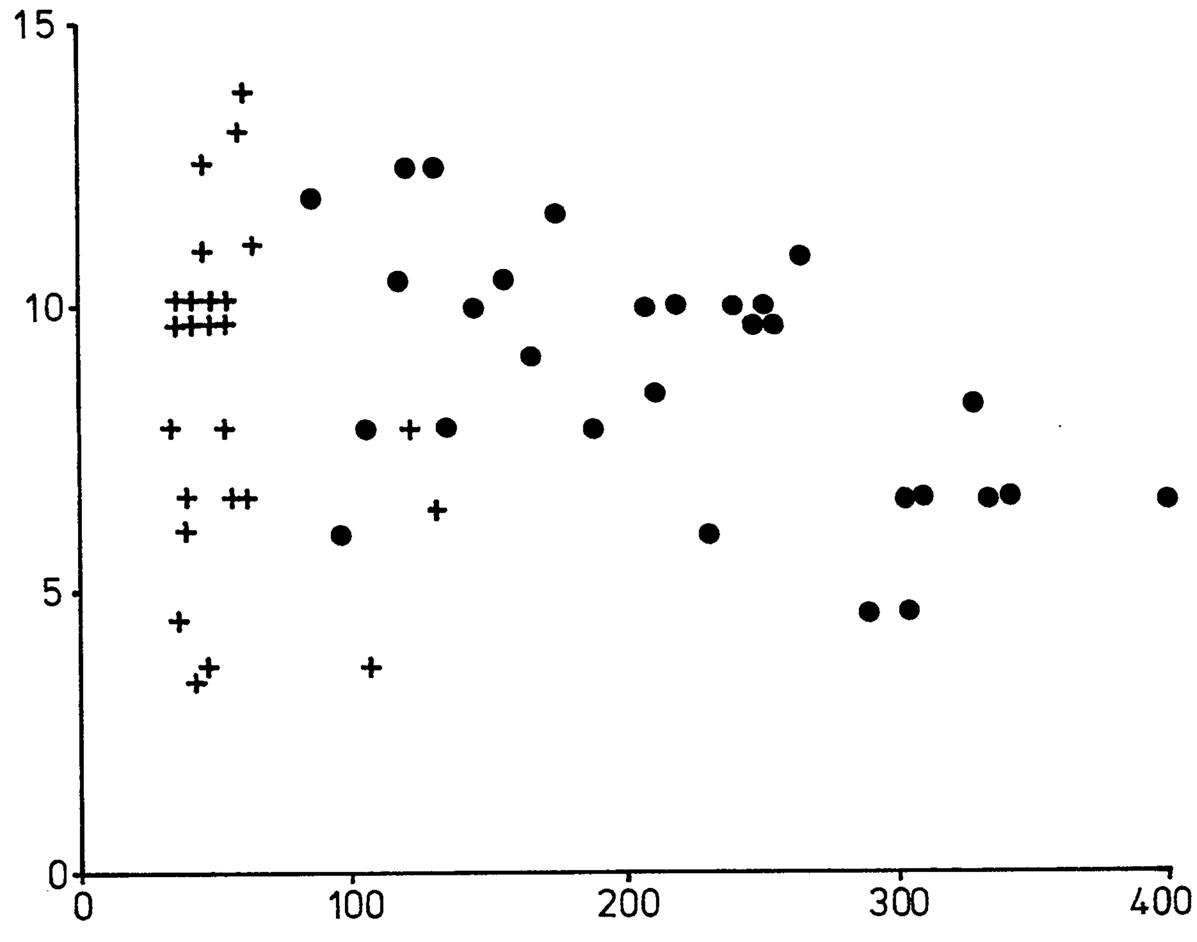


FIG. 2

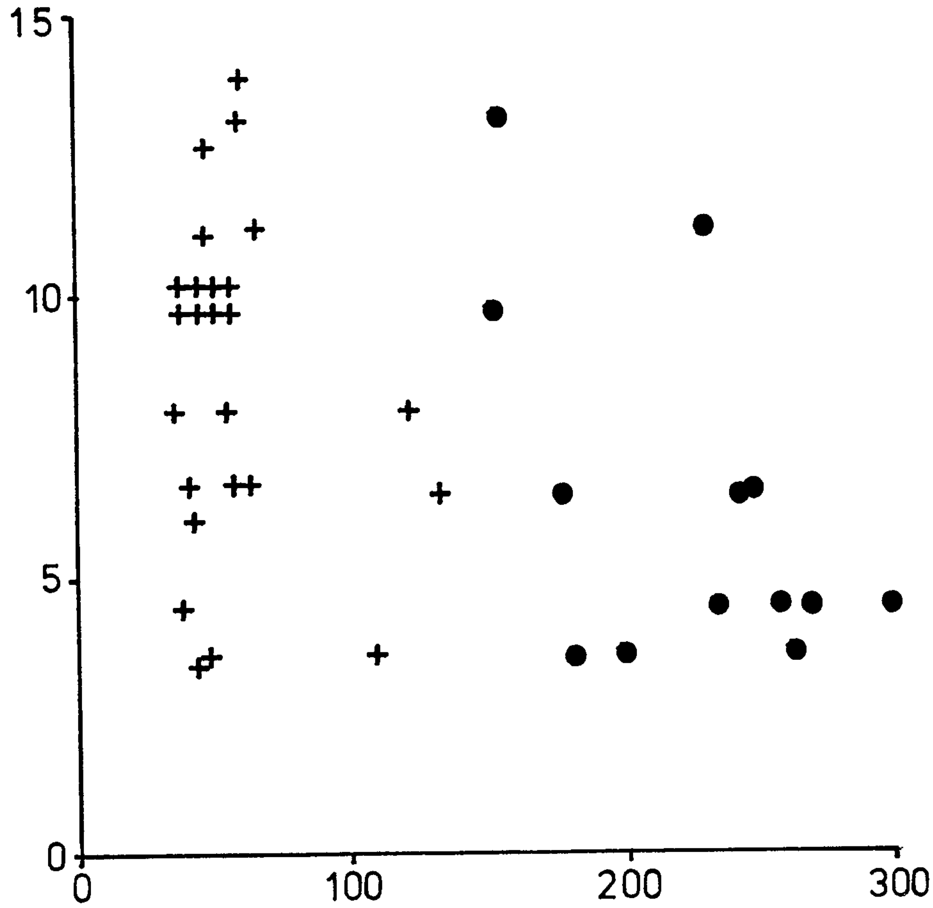


FIG. 3

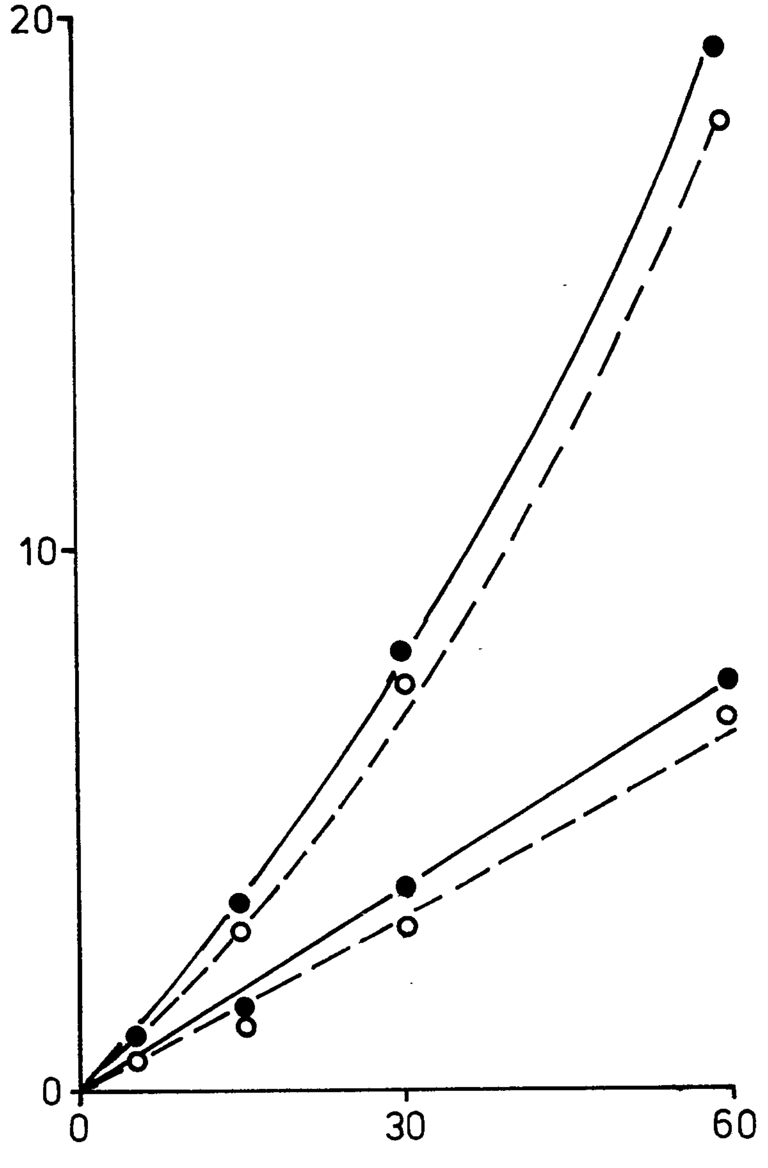


FIG. 4